# FASCICULE DE BREVET EUROPEEN

(11) **EP 0 755 668 B1**
(45) Date de publication et mention de la délivrance du brevet: **30.09.1998**
(21) Numéro de dépôt: 96401412.0
(22) Date de dépôt: 26.06.1996
(51) Int. Cl.: A61K 7/025, A61K 7/027, A61K 7/02, A61K 7/48

(54) **Composition sous forme de pâte souple obtenue par extrusion et procédé de préparation**
Durch extrudieren erhaltene, in Form einer weichen Paste vorliegende Zusammensetzung und Herstellungsverfahren
Composition in form of a supple paste obtained by extrusion and preparation process

(30) Priorité: 25.07.1995 FR 9509025
(43) Date de publication de la demande: 29.01.1997
(73) Titulaire: L'OREAL, 75008 Paris (FR)
(72) Inventeur: Roulier, Véronique, 75002 Paris (FR); Miguel-Colombel, Dolorès, 94240 L'Hay-les-Roses (FR)
(74) Mandataire: Dodin, Catherine

(56) Documents cités:
- EP-A- 0 602 905
- EP-A- 0 605 284

## Description

La présente invention a trait à une composition notamment cosmétique ou dermatologique, se présentant sous forme d'une pâte souple et pouvant être utilisée pour le soin et/ou le maquillage des lèvres et/ou de la peau. L'invention concerne également un procédé de préparation de cette composition.

EP-A-0 605 284 décrit une composition solide ou pâteuse de maquillage contenant une phase grasse et une phase pulvérulente et un procédé pour obtenir une telle composition par passage dans un mélangeur extrudeur.

Les compositions cosmétiques pouvant être appliquées sur la peau ou les lèvres comme produit de maquillage ou de soin, telles que les bases pour lèvres ou les rouges à lèvres par exemple, peuvent se présenter, de manière connue sous la forme d'un stick solide, par exemple un bâton de rouge à lèvres, ou sous la forme de pâte souple, applicable à l'aide d'un pinceau, par exemple.
Les compositions sous forme de stick comprennent généralement des cires en quantité importante, ainsi que des huiles, des pigments et/ou charges et, éventuellement, des additifs. Les compositions sous forme de pâte souple ne contiennent généralement pas, ou peu, de cires, ce qui permet de les prélever et de les appliquer aisément, étant donné qu'une quantité importante des cires conduirait à une composition de viscosité élevée, donc difficilement applicable.
Toutefois, les cires permettent d'apporter certaines propriétés intéressantes aux compositions les comprenant, en particulier des qualités d'onctuosité de la composition et des qualités de tenue et d'épaisseur du film déposé. Ainsi, il serait souhaitable de disposer de compositions sous forme de pâte souple pouvant comprendre des quantités importantes de cires.
D'autre part, il serait intéressant d'accentuer certaines des qualités cosmétiques des compositions sous forme de pâte souple; en particulier, on souhaiterait encore améliorer leur douceur lors de leur application sur les lèvres ou la peau, tout en apportant une certaine hydratation et un certain soin au support.
Ainsi, on souhaiterait pouvoir incorporer dans des compositions sous forme de pâte souple, des ingrédients hydratants ou de soin, tels que par exemple des alcools polyhydriques, ou des actifs notamment en dispersion ou en solution dans une phase aqueuse. Or, on a constaté que les alcools polyhydriques, et notamment la glycérine, tout comme les phases aqueuses gélifiées, sont incompatibles avec les corps gras d'une manière générale; en effet, on a également constaté qu'une composition comprenant des corps gras et de la glycérine ne se présente pas sous la forme d'une seule phase homogène, mais sous la forme de deux phases séparées, ce qui implique des problèmes de conservation au cours du temps.

Une des solutions pour pallier cet inconvénient est d'incorporer dans la composition, notamment lorsqu'elle se présente sous forme de stick, un tensioactif, qui permet de maintenir la composition sous forme d'une seule phase homogène. Toutefois, la composition obtenue peut présenter un certain caractère irritant. Il est également possible de choisir les corps gras en fonction de l'alcool polyhydrique, sachant que certains corps gras particuliers sont compatibles avec certains alcools polyhydriques, l'isoprène-glycol par exemple; toutefois, ceci implique une grande limitation au niveau des formulations envisageables.

La présente invention a pour but de pallier ces inconvénients, et propose une composition sous forme de pâte souple qui est particulièrement homogène, bien qu'elle comprenne d'une part des composants lipophiles tels que des corps gras et d'autre part des composants hydrophiles tels que des alcools polyhydriques et/ou une phase aqueuse.

Un objet de la présente invention est donc une composition extrudée sous forme d'une pâte souple, telle que définie dans la revendication 1.

Un autre objet de l'invention est un procédé de préparation de ladite composition dans lequel on effectue au moins une partie du procédé à l'aide d'un mélangeur-extrudeur avec malaxage pendant au moins une partie du refroidissement jusqu 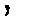 à la température ambiante.

Ainsi, d'une manière générale, le procédé selon l'invention permet d'obtenir une composition homogène à partir de deux phases incompatibles entre elles, l'une de ces phases étant lipophile et l'autre étant hydrophile.
Un avantage de l'invention est qu'elle permet la préparation de compositions homogènes de formulations variées, sans être tenu par la présence d'un certain type de corps gras par exemple.
D'autre part, la composition obtenue peut être stable et homogène bien qu'elle ne contienne pas de tensioactif.

La composition selon la présente invention est donc une pâte souple, dont on peut mesurer la viscosité, par opposition à la structure solide d'un bâton, ou stick,
dont on ne peut pas mesurer la viscosité. Ladite viscosité dynamique à 25°C est généralement comprise entre 3 et 35 Pa.s, mesurée avec un viscosimètre rotatif CONTRAVES TV équipé d'un mobile "MS-r4" à la fréquence de 60 Hz.

La composition selon l'invention comprend donc au moins un composant lipophile, qui peut être choisi, par exemple, parmi les constituants usuels d'une phase grasse tels que des cires, des huiles, des gommes et/ou des corps gras pâteux, hydrocarbonés et/ou siliconés, éventuellement volatils, seuls ou en mélange. Parmi les cires envisageables, seules ou en mélange, on peut citer les cires minérales telles que les cires microcristallines, la paraffine, le pétrolatum, la vaseline, l 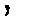 ozokérite, la cire de montan; les cires animales telles que la cire d 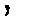 abeilles, la lanoline et ses dérivés; les cires végétales telles que les cires de Candellila, d'Ourrury, de Carnauba, du Japon, le beurre de cacao, les cires de fibres de lièges ou de canne à sucre; les huiles hydrogénés, les esters gras et les glycérides concrets à 25°C; les cires synthétiques, telles que les cires de polyéthylène et les cires obtenues par synthèse de Fischer-Tropsch; les cires de silicone; leurs mélanges.
Parmi les huiles envisageables, on peut citer, seules ou en mélange, les huiles minérales telles que l'huile de paraffine ou de vaseline; les huiles animales telles que le perhydrosqualène ou l'huile d'arara; les huiles végétales telles que l'huile d'amande douce, de calophyllum, de palme, de ricin, d'avocat, de jojoba, d'olive ou de germes de céréales; les huiles de silicone telles que le diméthylpolysiloxane; des esters d'acide lanolique, d'acide oléique, d'acide laurique, d'acide stéarique ou d'acide myristique par exemple; des alcools tels que l'alcool oléique, l'alcool linoléique ou linolénique, l 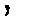 alcool isostéarique ou l'octyl dodécanol; des acétylglycérides, des octanoates, décanoates ou ricinoléates d'alcools ou de polyalcools; les huiles volatiles, telles que la cyclotétradiméthylsiloxane, la cyclopentadiméthylsiloxane, la cyclohexadiméthylsiloxane et le méthylhexyldiméthylsiloxane, ou les isoparaffines.
La composition peut comprendre 65-99% en poids de composant lipophile, qui peut être constitué de 8-40% en poids de cires et 60-92% en poids d'huiles.
Grâce au procédé utilisé pour préparer la présente composition, il est possible d'ajouter une quantité importante de cires dans la composition, par exemple de l'ordre de 15-25% en poids, sans entraîner de problème d'homogénéité ou de formation de grains. Ainsi, la composition obtenue ayant une teneur en cires élevée, supérieur à 10% en poids, est susceptible de former un film de bonne tenue.

La composition selon l'invention comprend également au moins un composant hydrophile, qui peut être un alcool polyhydrique et/ou qui peut se présenter sous la forme d'une phase aqueuse gélifiée.
L 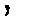 alcool polyhydrique peut être un composé ayant 2-8 atomes de carbone et 2-6 fonctions hydroxyles, tel que l 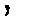 éthylène glycol, l'isoprène-glycol, le glycérol, le propane-1,2 diol, la diglycérine, l 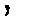 érythritol, l 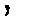 arabitol, l 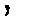 adonitol, le sorbitol et le dulcitol. L 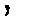 alcool polyhydrique peut également être un polyéther alcool de poids moléculaire moyen de 150-600, tel que le polyéthylène glycol 300 et la polyglycérine 500. On peut aussi employer un mélange d'alcools polyhydriques.
La phase aqueuse gélifiée peut être gélifiée par la présence de tout agent gélifiant connu de l'homme du métier.
On peut notamment citer comme agent gélifiant:
. les extraits d'algues tels que l'agar-agar, les carraghénanes et les alginates,
. les extraits de graines tels que les extraits de caroube ou de guar,
. les extraits de fruits, notamment la pectine,
. les exsudats de plantes, tels que la gomme arabique, l'adragante, la gomme de karaya et la gomme de ghatty,
. les dérivés cellulosiques, tels que la carboxyméthylcellulose,
. les gélifiant d'origine animale tels que la gélatine ou les caseinates,
. les exsudats de micro-organismes tels que la gomme xanthane,
. les gélifiants synthétiques tels que les dérivés de polymère acrylique (Carbomer, Carbopol, Pemulen) ou les dérivés du silicium (Lapointe, Lapomer ou Veegum).
L'agent gélifiant peut être présent dans la phase aqueuse à raison de 0,2-10% en poids par rapport à la phase aqueuse.
Le composant hydrophile peut présenter une viscosité de 1 à 250 poises, de préférence 30 à 250 poises. Le composant lipophile, quant à lui, peut présenter une viscosité supérieure ou inférieure à celle du composant hydrophile.
La composition peut comprendre 1-35% en poids de composant hydrophile, de préférence 3-15% en poids, ledit composant hydrophile pouvant également se présenter sous la forme d'un mélange de phase aqueuse gélifiée et d'alcool polyhydrique.

La composition peut également comprendre tout constituant usuellement utilisé dans le domaine d'application envisagé, et notamment des antioxydants, des parfums, des huiles essentielles, des conservateurs, des actifs cosmétiques, des hydratants, des vitamines, des acides gras essentiels, des sphingolipides, des filtres solaires, des tensioactifs, des colorants, des pigments, des nacres, des charges, des polymères. Selon la nature de ces constituants additionnels, on les ajoutera au composant lipophile ou au composant hydrophile lors de la préparation de la composition. Bien entendu l'homme du métier veillera à choisir ces éventuels constituants complémentaires, ainsi que leur quantité, de manière telle que les propriétés avantageuses de la composition selon l'invention ne soient pas, ou substantiellement pas, altérées par l'adjonction envisagée.

La composition selon l'invention peut se présenter sous la forme d'un produit de maquillage de la peau, en particulier sous la forme d'un fond de teint, d'un fard à joues ou à paupières, d'un mascara ou d'un eye liner, d'un rouge à lèvres ou d'une base pour lèvres, ou encore sous la forme d'un produit de soin de la peau, ou d'un produit solaire ou auto-bronzant, voire sous la forme d'un produit capillaire.
La composition selon l'invention permet l'obtention d'un film s 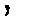 étalant facilement et uniformément, avec une certaine douceur. Le film obtenu présente une texture légère et reste confortable à porter tout au long de la journée. Il possède également une bonne tenue. La composition obtenue présente également de bonnes propriétés émollientes par application sur la peau.

La composition selon l'invention peut être préparée de manière avantageuse à l'aide d'un mélangeur-extrudeur.
On peut, par exemple, préparer un prémélange comprenant au moins une partie des différents constituants de la composition, dont ceux ayant une température de fusion élevée, chauffer ce prémélange à une température à laquelle il fond, puis ajouter le reste des constituants, en une ou plusieurs fois, et malaxer le mélange obtenu dans un mélangeur-extrudeur, pendant au moins une partie de son refroidissement jusqu'à température ambiante.
On peut également introduire la totalité des constituants en tête du mélangeur-extrudeur, à froid ou à chaud, puis éventuellement augmenter la température dudit mélange afin d'obtenir un mélange homogène, tout en malaxant et continuer le malaxage pendant au moins une partie du refroidissement jusqu'à température ambiante.
On a en effet constaté que ce procédé permet d 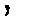 obtenir une composition se présentant sous forme de pâte souple et homogène, bien qu'elle contienne un composant hydrophile et un composant lipophile.
On peut effectuer l'étape de chauffage selon toute technique connue, et notamment directement dans l'extrudeur.
Les différents étapes du procédé peuvent être réalisées dans un ou plusieurs extrudeurs disposés à la suite les uns des autres, et de préférence dans un extrudeur bivis unique.
On a en effet constaté que la composition obtenue après extrusion présente une douceur particulière, et procure une certaine sensation de glissant lorsqu'elle est appliquée sur la peau, tout en évitant l'aspect et la sensation de gras huileux.
Les conditions dans lesquelles l'extrusion peut être effectuée sont décrites dans la demande de brevet FR94-00756.

L'invention est illustrée plus en détail dans les exemples suivants.

### Exemple 1

On prépare un rouge à lèvres sous forme de pâte souple, ayant la composition suivante:

| | |
|---|---|
| . huile de jojoba | 15 g |
| . huile de ricin | 10 g |
| . lanoline | 10 g |
| . cire d'abeilles | 10 g |
| . cire de polyéthylène | 15 g |
| . polybutène | 5 g |
| . polyglycérine | 20 g |
| . pigments et charges | 15 g |

On introduit séparément les phases hydrophile et lipophile dans un extrudeur bivis, à une température d'environ 85-95°C en entrée, et de 30°C en sortie. La vitesse des vis est fixée à environ 400 tours/minute.
On obtient en sortie une pâte souple de viscosité égale à 13 Pa.s, se présentant sous forme d'une seule phase stable et homogène, et pouvant être prélevée à l'aide d'un pinceau pour son application. Cette composition permet l'obtention d'un film homogène et s'étalant facilement et présentant beaucoup de douceur. Le film obtenu présente également une texture légère et est confortable à porter tout au long de la journée.

### Exemple 2

On prépare un rouge à lèvres sous forme de pâte souple, ayant la composition suivante:

| | |
|---|---|
| . huile de jojoba | 20 g |
| . huile de vaseline | 20 g |
| . lanoline | 20 g |
| . cire de Candelilla | 7 g |
| . cire de polyéthylène | 10 g |
| . isoprène-glycol | 1 g |
| . pigments | 12 g |
| . charges (talc et poudre de Nylon) | 10 g |

On prépare la composition selon l'exemple 1. On obtient une composition homogène, présentant de bonnes propriétés cosmétiques.

### Exemple 3

On prépare, selon l'exemple 1, une base traitante pour les lèvres, sous forme de pâte souple, ayant la composition suivante:

| | |
|---|---|
| . huile de vaseline | 22 g |
| . lanolate d'isopropyle | 20 g |
| . lanoline | 20 g |
| . cire microcristalline | 15 g |
| . cire de Carnauba | 10 g |
| . glycérine | 10 g |
| . acétate de tocophérol | 2 g |
| . octylméthoxycinnamate | 1 g |

La composition se présente sous forme d'une seule phase homogène et possède de bonnes propriétés cosmétiques, une certaine douceur à l'application et de bonne propriétés hydratantes.

### Exemple 4

On prépare selon l'exemple 1, une composition comprenant :

| | |
|---|---|
| . huile de vaseline | 22 g |
| . lanolate d'isopropyle | 20 g |
| . lanoline | 20 g |
| . cire microcristalline | 15 g |
| . cire de Carnauba | 10 g |
| . phase aqueuse gélifiée (0,1 g de Carbopol + 9,9 g d'eau) | 10 g |
| . acétate de tocophérol | 2 g |
| . octylméthoxycinnamate | 1 g |

On obtient une composition se présentant sous forme d'une seule phase stable et homogène. Elle permet l'obtention d'un film homogène et s 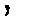 étalant facilement.

### Exemple 5

On prépare selon l'exemple 1, une composition comprenant :

| | |
|---|---|
| . huile de vaseline | 17 g |
| . lanolate d'isopropyle | 15 g |
| . lanoline | 15 g |
| . cire microcristalline | 15 g |
| . cire de Carnauba | 10 g |
| . acétate de tocophérol | 2 g |
| . octylméthoxycinnamate | 1 g |
| . phase aqueuse gélifiée (1,25g carboxyméthylcellulose + 23,75g eau) | 25 g |

On obtient une composition se présentant sous forme d'une seule phase stable et homogène, ayant de bonnes propriétés cosmétiques.

### Exemple 6: exemple comparatif

On prépare un rouge à lèvre sous forme de pâte souple classique ayant la composition suivante :

| | |
|---|---|
| . huile de vaseline | 62 g |
| . lanolinate d'isopropyle | 10 g |
| . lanoline | 10 g |
| . cire microcristalline | 2 g |
| . glycérine | 5 g |
| . pigments | 5 g |

On mélange les différents ingrédients à 100°C à l'aide d'un agitateur de type Moritz. On obtient un mélange hétérogène présentant deux phases. Il n'est pas possible, avec ce procédé d'obtenir une dispersion homogène. Si l'on augmente le taux de cire, par exemple à 8%, il n'est pas non plus possible d'obtenir un mélange homogène.

## Revendications

1. Composition extrudée, comprenant un composant lipophile et un composant hydrophile, caractérisée en ce quelle se présente sous forme de pâte souple et d'une seule phase homogène, et est obtenue par un procédé comportant une étape de malaxage pendant au moins une partie du refroidissement jusqu'à la température ambiante.

2. Composition selon la revendication 1, dans laquelle le composant hydrophile est choisi parmi les alcools polyhydriques, les phases aqueuses gélifiées, ou leurs mélanges.

3. Composition selon la revendication 2, dans laquelle l 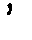 alcool polyhydrique est choisi parmi les composés ayant 2-8 atomes de carbone et 2-6 fonctions hydroxyles, tel que l 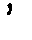 éthylène glycol, l'isoprène-glycol, le glycérol, le propane-1,2 diol, la diglycérine, l 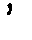 érythritol, l 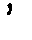 arabitol, l 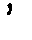 adonitol, le sorbitol et le dulcitol, et/ou parmi les polyéthers alcool de poids moléculaire moyen de 150-600, tel que le polyéthylène glycol 300 et la polyglycérine 500; ou leurs mélanges.

4. Composition selon la revendication 2, dans laquelle la phase aqueuse est gélifiée à l'aide d'un agent gélifiant choisi parmi :
. les extraits d'algues tels que l'agar-agar, les carraghénanes et les alginates,
. les extraits de graines tels que les extraits de caroube ou de guar,
. les extraits de fruits, notamment la pectine,
. les exsudats de plantes, tels que la gomme arabique, l'adragante, la gomme de karaya et la gomme de ghatty,
. les dérivés cellulosiques, tels que la carboxyméthylcellulose,
. les gélifiant d'origine animale tels que la gélatine ou les caseinates,
. les exsudats de micro-organismes tels que la gomme xanthane,
. les gélifiants synthétiques tels que les dérivés de polymère acrylique ou les dérivés du silicium, et
. leurs mélanges.

5. Composition selon la revendication 4 dans laquelle l'agent gélifiant est présent dans la phase aqueuse à raison de 0,2-10% en poids.

6. Composition selon l'une des revendications précédentes, dans laquelle le composant hydrophile présente une viscosité de 0.1 à 25 Pa.s (1 à 250 poises), de préférence 3.0 à 25 Pa.s (30 à 250 poises).

7. Composition selon l'une des revendications précédentes, dans laquelle le composant lipophile est choisi parmi les cires, les huiles, les gommes et/ou les corps gras pâteux, hydrocarbonés et/ou siliconés, éventuellement volatils, seuls ou en mélange.

8. Composition selon l'une des revendications précédentes, comprenant 1-35% en poids de composant hydrophile, de préférence 3-15% en poids.

9. Composition selon l'une des revendications précédentes, comprenant 65-99% en poids de composant lipophile, qui peut être constitué de 8-40% en poids de cires et 60-92% en poids d'huiles.

10. Composition selon l'une des revendications précédentes, dans laquelle le composant lipophile comprend au moins 10% en poids par rapport à la composition, de préférence 15-25% en poids de cires.

11. Composition selon l'une des revendications précédentes, ayant une viscosité dynamique à 25°C comprise entre 3 et 35 Pa.s, mesurée avec un viscosimètre rotatif CONTRAVES TV équipé d'un mobile "MS-r4" à la fréquence de 60 Hz.

12. Composition selon l'une des revendications précédentes, ne comprenant pas de tensioactif.

13. Composition selon l 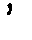 une des revendications précédentes, se présentant sous la forme d 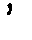 un produit de maquillage de la peau, tel qu'un fond de teint, un fard à joues ou à paupières, un mascara, un eye liner, un rouge à lèvres ou une base pour lèvres, ou sous la forme d'un produit de soin de la peau, ou d'un produit solaire ou auto-bronzant, ou d'un produit capillaire.

14. Procédé de préparation d'une composition selon l'une des revendications 1 à 13, dans lequel on effectue au moins une partie du procédé à l'aide d'un mélangeur-extrudeur et on malaxe le mélange des différents constituants de la composition pendant au moins une partie de son refroidissement jusqu'à la température ambiante.

15. Procédé selon la revendication 14, dans lequel on prépare un prémélange comprenant au moins une partie des différents constituants de la composition, dont ceux ayant une température de fusion élevée, on chauffe ce prémélange à une température à laquelle il fond, puis on ajoute le reste des constituants, en une ou plusieurs fois, et l'on malaxe le mélange obtenu dans un mélangeur-extrudeur, pendant au moins une partie de son refroidissement jusqu'à température ambiante.

16. Procédé selon la revendication 14, dans lequel on introduit la totalité des constituants en tête du mélangeur-extrudeur, à froid ou à chaud, puis éventuellement on augmente la température dudit mélange afin d'obtenir un mélange homogène, tout en malaxant et on continue le malaxage pendant au moins une partie du refroidissement jusqu'à température ambiante.

17. Procédé selon l'une des revendications 14 à 16, dans lequel l'étape de chauffage est effectuée dans un mélangeur-extrudeur.

18. Procédé selon l'une des revendications 14 à 17, dans lequel les différentes étapes du procédé sont réalisées dans un ou plusieurs mélangeurs-extrudeurs disposés à la suite les uns des autres.

19. Procédé selon l'une des revendications 14 à 18, dans lequel les différentes étapes du procédé sont réalisées dans un mélangeur-extrudeur bivis unique.

## Claims

1. Extruded composition comprising a lipophilic component and a hydrophilic component, characterized in that it is in the form of a smooth paste and of a single homogeneous phase, and is obtained by a process including a blending step during at least part of the period of cooling to room temperature.

2. Composition according to Claim 1, in which the hydrophilic component is chosen from polyhydric alcohols and gelled aqueous phases, or mixtures thereof.

3. Composition according to Claim 2, in which the polyhydric alcohol is chosen from compounds having 2-8 carbon atoms and 2-6 hydroxyl functions, such as ethylene glycol, isoprene-glycol, glycerol, 1,2-propanediol, diglycerol, erythritol, arabitol, adonitol, sorbitol and dulcitol, and/or from polyether alcohols with an average molecular weight of 150-600, such as polyethylene glycol 300 and polyglycerol 500; or mixtures thereof.

4. Composition according to Claim 2, in which the aqueous phase is gelled using a gelling agent chosen from:
• alga extracts such as agar-agar, carrageenans and alginates,
• seed extracts such as extracts of carob or of guar,
• fruit extracts, in particular pectin,
• plant exudates, such as gum arabic, gum tragacanth, karaya gum and ghatti gum,
• cellulose derivatives such as carboxymethyl cellulose,
• gelling agents of animal origin such as gelatin or caseinates,
• exudates of microorganisms such as xanthan gum,
• synthetic gelling agents such as acrylic polymer derivatives or silicon derivatives, and
• mixtures thereof.

5. Composition according to Claim 4, in which the gelling agent is present in the aqueous phase in a proportion of 0.2-10 % by weight.

6. Composition according to one of the preceding claims, in which the hydrophilic component has a viscosity of from 0.1 to 25 Pa.s (1 to 250 poises), preferably 3.0 to 25 Pa.s (30 to 250 poises).

7. Composition according to one of the preceding claims, in which the lipophilic component is chosen from waxes, oils, gums and/or fatty substances which are pasty, hydrocarbon-containing and/or silicone-containing, and possibly volatile, alone or as a mixture.

8. Composition according to one of the preceding claims, comprising 1-35 % by weight of hydrophilic component, preferably 3-15 % by weight.

9. Composition according to one of the preceding claims, comprising 65-99 % by weight of lipophilic component, which may consist of 8-40 % by weight of waxes and 60-92 % by weight of oils.

10. Composition according to one of the preceding claims, in which the lipophilic component comprises at least 10 % by weight relative to the composition, preferably 15-25 % by weight, of waxes.

11. Composition according to one of the preceding claims, having a dynamic viscosity at 25°C of between 3 and 35 Pa s, measured with a Contraves TV rotary viscometer equipped with an "MS-r4" rotor at a frequency of 60 Hz.

12. Composition according to one of the preceding claims, which comprises no surfactant.

13. Composition according to one of the preceding claims, in the form of a make-up product for the skin, such as a foundation, a blusher, an eyeshadow, a mascara, an eyeliner, a lipstick or a base for the lips, or in the form of a care product for the skin or an antisun or self-tanning product, or in the form of a hair product.

14. Process for the preparation of a composition according to one of Claims 1 to 13, in which at least part of the process is carried out using a mixer-extruder, and the mixture of the various constituents of the composition are blended during at least part of its period of cooling to room temperature.

15. Process according to Claim 14, in which a premix is prepared comprising at least some of the various constituents of the composition, including those having a high melting point, this premix is heated to a temperature at which it melts, and then the rest of the constituents are added, in one or more portions, and the mixture obtained is blended in a mixer-extruder, during at least part of its period of cooling to room temperature.

16. Process according to Claim 14, in which all of the constituents are introduced into the top of the mixer-extruder, under hot or cold conditions, then optionally the temperature of the said mixture is increased so as to obtain a homogeneous mixture, while at the same time blending, and the blending is continued for at least part of the period of cooling to room temperature.

17. Process according to one of Claims 14 to 16, in which the heating step is carried out in a mixer-extruder.

18. Process according to one of Claims 14 to 17, in which the various steps of the process are performed in one or more mixer-extruders arranged one after the other.

19. Process according to one of Claims 14 to 18, in which the various steps of the process are performed in a single twin-screw mixer-extruder.

## Patentansprüche

1. Extrudierte Zusammensetzung, die eine lipophile Komponente und eine hydrophile Komponente enthält, dadurch gekennzeichnet, daß sie in Form einer weichen Paste und als einzige homogene Phase vorliegt und daß sie durch ein Verfahren hergestellt wird, das einen Knetschritt während mindestens eines Teils der zum Abkühlen auf Umgebungstemperatur erforderlichen Zeit umfaßt.

2. Zusammensetzung nach Anspruch 1, worin die hydrophile Komponente unter den mehrwertigen Alkoholen, den gelierten, wässerigen Phasen oder deren Gemischen ausgewählt ist.

3. Zusammensetzung nach Anspruch 2, worin der mehrwertige Alkohol unter den Verbindungen mit 2 bis 8 Kohlenstoffatomen und 2 bis 6 Hydroxygruppen, wie Ethylenglykol, Isoprenglykol, Glycerin, 1,2-Propandiol, Diglycerin, Erythrit, Arabit, Adonit, Sorbit und Dulcit, und/oder den Polyetheralkoholen mit einem mittleren Molekülgewicht von 150 bis 600, wie Polyethylenglykol 300 und Polyglycerin 500, oder den Gemischen dieser Verbindungen ausgewählt ist.

4. Zusammensetzung nach Anspruch 2, worin die wässerige Phase mit einem Gelbildner geliert ist, der ausgewählt ist unter:
- Algenextrakten, wie Agar-Agar, Carrageenanen und Alginaten,
- Samenextrakten, wie Johannisbrotkernextrakten oder Guarextrakten,
- Fruchtextrakten, insbesondere Pectin,
- Pflanzenexsudaten, wie Gummi arabicum, Tragant, Karaya-Gummi und Ghatti gummi,
- Cellulosederivaten, wie Carboxymethylcellulose,
- Gelbildnern tierischer Herkunft, wie Gelatine oder Caseinaten,
- Exsudaten von Mikroorganismen, wie Xanthangummi,
- synthetischen Gelbildnern, wie den Derivaten von Acrylpolymeren oder Siliciumderivaten und
- den Gemischen dieser Verbindungen.

5. Zusammensetzung nach Anspruch 4, worin der Gelbildner in der wässerigen Phase in einem Mengenanteil von 0,2 bis 10 Gew.-% vorliegt.

6. Zusammensetzung nach einem der vorhergehenden Ansprüche, worin die hydrophile Komponente eine Viskosität von 0,1 bis 25 Pa·s (1 bis 250 Poise), vorzugsweise von 3,0 bis 25 Pa·s (30 bis 250 Poise), aufweist.

7. Zusammensetzung nach einem der vorhergehenden Ansprüche, worin die lipophile Komponente unter Wachsen, Ölen, Gummen und/oder pastösen Fettsubstanzen, die kohlenwasserstoffhaltig und/oder siliconhaltig und gegebenenfalls flüchtig sind und allein oder im Gemisch verwendet werden, ausgewählt ist.

8. Zusammensetzung nach einem der vorhergehenden Ansprüche, die 1 bis 35 Gew.-%, vorzugsweise 3 bis 15 Gew.-%, hydrophile Komponente enthält.

9. Zusammensetzung nach einem der vorhergehenden Ansprüche, die 65 bis 99 Gew.-% lipophile Komponente, welche zu 8 bis 40 Gew.-% aus Wachsen und zu 60 bis 92 Gew.-% aus Ölen bestehen kann, enthält.

10. Zusammensetzung nach einem der vorhergehenden Ansprüche, worin die lipophile Komponente mindestens 10 Gew.-% Wachse, bezogen auf die Zusammensetzung, und vorzugsweise 15 bis 25 Gew.-% Wachse enthält.

11. Zusammensetzung nach einem der vorhergehenden Ansprüche, die eine dynamische Viskosität bei 25 °C im Bereich von 3 bis 35 Pa·s aufweist, die mit einem Rotationsviskosimeter CONTRAVES TV, das mit einem beweglichen Körper 'MS-r4' ausgestattet ist, bei einer Frequenz von 60 Hz bestimmt wurde.

12. Zusammensetzung nach einem der vorhergehenden Ansprüche, die keinen grenzflächenaktiven Stoff enthält.

13. Zusammensetzung nach einem der vorhergehenden Ansprüche, die in Form eines Schminkproduktes für die Haut, wie einem Make-up, einem Wangenrouge oder einem Lidschatten, einer Mascara, einem Eyeliner, einem Lippenstift oder einer Grundmasse für die Lippen, oder in Form eines Pflegeproduktes für die Haut, eines Sonnenschutzproduktes, eines Selbstbräuners oder eines Produktes zur Haarpflege vorliegt.

14. Verfahren zur Herstellung einer Zusammensetzung nach einem der Ansprüche 1 bis 13, worin mindestens ein Teil des Verfahrens mit einem Mischer-Extruder durchgeführt wird und worin das Gemisch der verschiedenen Bestandteile der Zusammensetzung während mindestens eines Teil der zum Abkühlen auf Umgebungstemperatur erforderlichen Zeit geknetet wird.

15. Verfahren nach Anspruch 14, worin ein Vorgemisch hergestellt wird, das mindestens einen Teil der verschiedenen Bestandteile der Zusammensetzung, darunter die Bestandteile mit einer hohen Schmelztemperatur, umfaßt, woraufhin dieses Vorgemisch auf eine Temperatur erwärmt wird, bei der es schmilzt, dann die restlichen Bestandteile auf einmal oder auf mehrere Male zugegeben werden und das erhaltene Gemisch in einem Mischer-Extruder während mindestens eines Teils der zum Abkühlen auf Umgebungstemperatur erforderlichen Zeit geknetet wird.

16. Verfahren nach Anspruch 14, worin sämtliche Bestandteile in der Kälte oder in der Wärme am Kopf des Mischer-Extruders eingebracht werden, woraufhin unter Kneten gegebenenfalls die Temperatur dieses Gemisches erhöht wird, um ein homogenes Gemisch zu erhalten, wobei das Kneten während mindestens eines Teils der zum Abkühlen auf Umgebungstemperatur erforderlichen Zeit fortgesetzt wird.

17. Verfahren nach einem der Ansprüche 14 bis 16, worin der Schritt des Erwärmens in einem Mischer-Extruder durchgeführt wird.

18. Verfahren nach einem der Ansprüche 14 bis 17, worin die verschiedenen Schritte des Verfahrens in einem oder mehreren Mischer-Extrudern, die hintereinander angeordnet sind, durchgeführt wird.

19. Verfahren nach einem der Ansprüche 14 bis 18, worin die verschiedenen Schritte des Verfahrens in einem einzigen Mischer-Extruder mit Doppelschnecke durchgeführt werden.
